# EUROPÄISCHE PATENTSCHRIFT

(11) **EP 0 726 258 B1**
(45) Veröffentlichungstag und Bekanntmachung des Hinweises auf die Patenterteilung: **29.05.2002**
(21) Anmeldenummer: 96101303.4
(22) Anmeldetag: 31.01.1996
(51) Int. Cl.: C07D 249/12, C07C 281/02, C07C 281/04

(54) **Verfahren und neue Zwischenprodukte zur Herstellung von Triazolinonen**
Process and intermediates for the preparation of triazolinones
Procédé et produits intermédiaires pour la préparation de triazolinones

(30) Priorität: 13.02.1995 DE 19504627
(43) Veröffentlichungstag der Anmeldung: 14.08.1996
(60) Teilanmeldung: 01126454.6
(73) Patentinhaber: BAYER AG, 51368 Leverkusen (DE)
(72) Erfinder: Wroblowsky, Heinz-Jürgen, D-40764 Langenfeld (DE)

(56) Entgegenhaltungen:
- EP-A- 0 577 394
- WO-A-94/19323
- DE-A- 2 336 827
- FR-A- 2 123 205
- US-A- 4 282 169
- J. HETEROCYCLIC CHEM., Bd. 22, 1985, Seiten 1121-1126, XP000196036 G.D. MADDING ET AL:
- LIEBIGS ANN. CHEM., 1974, Seiten 504-522, XP000569702 H. RÖCHLING ET AL:
- SYNTHESIS, 1991, Seiten 350-352, XP000196018 C. ASTORGA ET AL:

## Beschreibung

Die Erfindung betrifft ein neues Verfahren und neue Zwischenprodukte zur Herstellung von weitgehend bekannten Triazolinonen, welche als Zwischenprodukte zur Herstellung von Herbiziden und Insektiziden verwendet werden können.

Es ist bekannt, dass man bestimmte substituierte Triazolinone, wie z.B. die Verbindung 4-Methyl-5-trifluormethyl-2,4-dihydro-3H-1,2,4-triazol-3-on erhält, wenn man entsprechende Triazolinthione, wie z.B. die Verbindung 4-Methyl-5-trifluormethyl-2,4-dihydro-3H-1,2,4-triazol-3-thion, zunächst mit einem Alkylierungsmittel, wie z.B. Methyliodid, in Gegenwart eines Säurebindemittels, wie z.B. Natriummethylat umsetzt, das hierbei gebildete Alkylthiotriazolderivat auf übliche Weise isoliert, dann mit Hydrogenperoxid in Gegenwart von Essigsäure erhitzt, nach Abkühlen neutralisiert und auf übliche Weise aufarbeitet (vgl. US-P 3780052 - Example 2).

Weiter ist bekannt, dass die oben genannte Verbindung 4-Methyl-5-trifluormethyl-2,4-dihydro-3H-1,2,4-triazol-3-on auch durch Erhitzen von 1-Trifluoracetyl-4-methylsemicarbazid auf 160°C bis 180°C, anschließende Extraktion mit Essigsäureethylester und Säulenchromatographie erhalten werden kann (vgl. US-P 3780052 - Example 3).

Bei den beiden angegebenen Synthesemethoden sind jedoch Ausbeute und Qualität der erhaltenen Produkte sehr unbefriedigend.

Weitere Verfahren zur Herstellung von Triazolinonen sind Gegenstand vorgängiger, jedoch nicht vorveröffentlichter Anmeldungen (vgl. DE-4 339 412 und DE-4 342 190).

Gegenstand der vorliegenden Anmeldung ist ein neues Verfahren zur Herstellung von Triazolinonen der allgemeinen Formel (I) 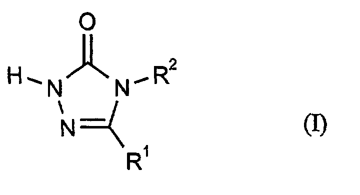 in welcher
- R¹: für zweifach oder dreifach durch Fluor substituiertes Methyl steht und
- R²: für jeweils gegebenenfalls durch Fluor, Chlor, Cyano, Methoxy oder Ethoxy substituiertes Methyl, Ethyl, n- oder i-Propyl, n-, i- oder s-Butyl, Allyl, Propargyl, Methoxy, Ethoxy, n- oder i-Propoxy, n-, i- oder s-Butoxy, Methylamino, Ethylamino, n- oder i-Propylamino, n-, i- oder s-Butylamino, Dimethylamino oder Diethylamino oder für jeweils gegebenenfalls durch Fluor, Chlor, Brom, Cyano, Methyl, Ethyl, n- oder i-Propyl, Methoxy-carbonyl oder Ethoxycarbonyl substituiertes Cyclopropyl, Cyclobutyl, Cyclopentyl, Cyclohexyl, Phenyl oder Benzyl steht,
dadurch gekennzeichnet, daß man Hydrazincarbonsäureester der allgemeinen Formel (II) 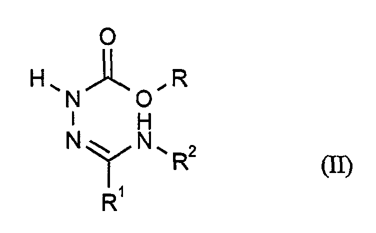 in welcher
- R¹ und R²: die oben angegebenen Bedeutungen haben und
- R: für gegebenenfalls durch Halogen oder C₁-C₄-Alkoxy substituiertes Alkyl mit 1 bis 4 Kohlenstoffatomen oder für gegebenenfalls durch Halogen oder C₁-C₄-Alkyl substituiertes Phenyl steht,
- und/oder zu den Verbindungen der Formel (II) tautomere Verbindungen -
in Gegenwart eines basischen Reaktionshilfsmittels und in Gegenwart eines Verdünnungsmittels bei Temperaturen zwischen 0°C und 150°C umsetzt.

Überraschenderweise können nach dem erfindungsgemäßen Verfahren die Triazolinone der allgemeinen Formel (I) auf einfache Weise in sehr hohen Ausbeuten erhalten werden, wobei die Substituenten breit variiert werden können.

Sicherheitstechnische Probleme, wie die Verwendung von Hydrogenperoxid in älteren Verfahren, und größere Abwasserprobleme, wie sie bei schwefelhaltigen Abwässern in älteren Verfahren auftreten, werden dabei vermieden.

Das erfindungsgemäße Verfahren stellt somit eine wertvolle Bereicherung des Standes der Technik dar.

Die bei den Restedefinitionen genannten Kohlenwasserstoffreste, wie Alkyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im Allgemeinen für Fluor, Chlor, Brom oder Iod, vorzugsweise für Fluor, Chlor oder Brom, insbesondere für Fluor oder Chlor.

Die oben aufgeführten allgemeinen Restedefinitionen gelten sowohl für die Endprodukte der Formel (I) als auch entsprechend für die jeweils zu Herstellung benötigten Ausgangsstoffe bzw. Zwischenprodukte.

Verwendet man beispielsweise 2-(2,2-Difluor-1-ethylimino-ethyl)-hydrazin-1-carbonsäure-ethylester als Ausgangsstoff, so kann der Reaktionsablauf beim erfindungsgemäßen Verfahren durch das folgende Formelschema skizziert werden:

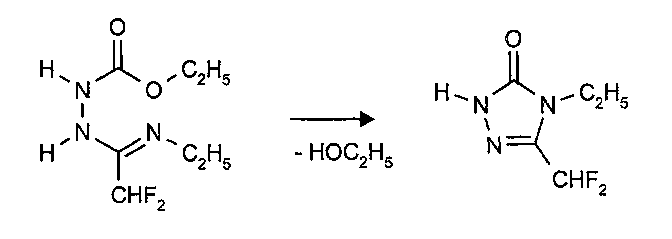

Die beim erfindungsgemäßen Verfahren zur Herstellung der Verbindungen der allgemeinen Formel (I) als Ausgangsstoffe zu verwendenden Hydrazincarbonsäureester sind durch die Formel (II) allgemein definiert. In der Formel (II) haben R¹ und R² diejenigen Bedeutungen, die bereits oben im Zusammenhang mit der Beschreibung der Verbindungen der Formel (I) für R¹ und R² angegeben wurden;

Die Hydrazincarbonsäureester der Formel (II) sind noch nicht aus der Literatur bekannt; sie sind als neue Stoffe Gegenstand der vorliegenden Anmeldung.

Man erhält die neuen Verbindungen der allgemeinen Formel (II), wenn man entsprechende Hydrazincarbonsäureester der allgemeinen Formel (III) 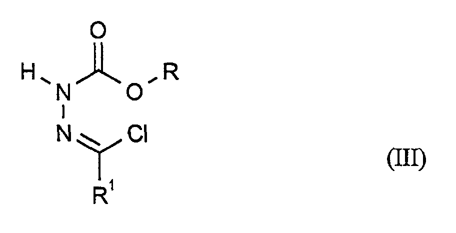 in welcher
- R und R¹: die oben angegebenen Bedeutungen haben,
mit Aminoverbindungen der allgemeinen Formel (IV)

H₂N-R² (IV)

in welcher
- R²: die oben angegebene Bedeutung hat,
gegebenenfalls in Gegenwart eines Säureakzeptors, wie z.B. Triethylamin und gegebenenfalls in Gegenwart eines Verdünnungsmittels, wie z.B. Acetonitril oder Dioxan, bei Temperaturen zwischen 0°C und 100°C umsetzt (vgl. die Herstellungsbeispiele).

Die als Vorprodukte zu verwendenden Hydrazincarbonsäureester sind durch die Formel (III) allgemein definiert. In der Formel (III) haben R¹ und R diejenigen Bedeutungen, die bereits oben im Zusammenhang mit der Beschreibung der Verbindungen der Formel (I) bzw. der Formel (II) für R¹ und R angegeben wurden.

Die Hydrazincarbonsäureester der Formel (III) sind noch nicht aus der Literatur bekannt.

Man erhält die neuen Verbindungen der allgemeinen Formel (III), wenn man entsprechende Hydrazincarbonsäureester der allgemeinen Formel (V) 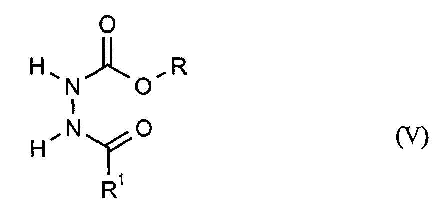 in welcher
- R und R¹: die oben angegebenen Bedeutungen haben,
mit einem Sulfonsäurechlorid, wie z.B. Methan-, Ethan-, Benzol- oder p-Toluol-sulfonsäurechlorid, gegebenenfalls in Gegenwart eines Säureakzeptors, wie z.B. Triethylamin oder N,N-Dimethyl-benzylamin, und gegebenenfalls in Gegenwart eines Verdünnungsmittels, wie z.B. Dimethoxyethan, Dioxan, Tetrahydrofuran, Methylenchlorid, Essigsäureethylester, Toluol, Chlorbenzol, Aceton, Methylethylketon, Methyl-isopropylketon, Methyl-isobutylketon, Acetonitril, Propionitril oder Butyronitril, bei Temperaturen zwischen 0°C und 100°C umsetzt (vgl. die Herstellungsbeispiele).

Die hierbei als Vorprodukte zu verwendenden Hydrazincarbonsäureester sind durch die Formel (V) allgemein definiert. In der Formel (V) haben R¹ und R diejenigen Bedeutungen, die bereits oben im Zusammenhang mit der Beschreibung der Verbindungen der Formel (I) bzw. der Formel (II) für R¹ und R angegeben wurden.

Die Hydrazincarbonsäureester der Formel (V) sind bekannt und/oder können nach bekannten Verfahren hergestellt werden (vgl. Synthesis 1991, 350-352; FR- 2 123 205).

Noch nicht aus der Literatur bekannt und als neue Verbindungen Gegenstand der vorliegenden Anmeldung sind die Hydrazincarbonsäureester der Formel (Va), 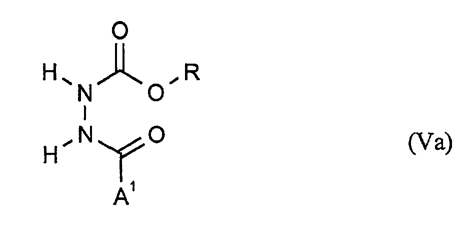 in welcher
- A¹: für Difluormethyl oder Trifluormethyl steht und
- R: die oben angegebene Bedeutung hat.

Man erhält die neuen Hydrazincarbonsäureester der Formel (Va), wenn man Hydrazincarbonsäureester der Formel (VI) 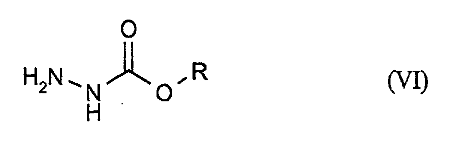 in welcher
- R: die oben angegebene Bedeutung hat,
mit Carbonsäuren oder deren Derivaten der allgemeinen Formel (VII)

A¹-CO-X (VII)

in welcher
- A¹: die oben angegebene Bedeutung hat und
- X: für Hydroxy, Halogen (insbesondere Fluor oder Chlor) oder die Gruppierung -O-CO-A¹ steht,
gegebenenfalls in Gegenwart eines Verdünnungsmittels, wie z.B. Diethylether, Acetonitril, Propionitril, Butyronitril, Chlorbenzol, Xylol oder Toluol, bei Temperaturen zwischen 0°C und 150°C umsetzt (vgl. die Herstellungsbeispiele).

Die Vorprodukte der Formeln (VI) und (VII) sind bekannte Synthesechemikalien.

Das erfindungsgemäße Verfahren wird in Gegenwart eines basischen Reaktionshilfsmittels durchgeführt. Als solche kommen alle üblichen anorganischen oder organischen Basen in Frage. Hierzu gehören beispielsweise Alkalimetall- oder Erdalkalimetall- -hydride, -hydroxide, -amide, -alkoholate, -acetate, -carbonate oder -hydrogencarbonate, wie beispielsweise Lithium-, Natrium-, Kalium- oder Calcium-hydrid, Lithium-, Natrium-, Kalium- oder Calcium-hydroxid, Lithium-, Natrium- oder Kalium-amid, Natrium- oder Kalium-methylat, Natrium- oder Kalium-ethylat, Natrium- oder Kaliumpropylat, Aluminiumisopropylat, Natrium- oder Kalium-tert-butylat, Natrium- oder Kalium-hydroxid, Ammoniumhydroxid, Natrium-, Kalium- oder Calcium-acetat, Ammoniumacetat, Natrium-, Kalium- oder Calcium-carbonat, Ammoniumcarbonat, Natrium- oder Kalium-hydrogencarbonat, sowie basische organische Stickstofiverbindungen, wie Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethylanilin, N,N-Dimethyl-benzylamin, Pyridin, 2-Methyl-, 3-Methylund 4-Methyl-pyridin, 2,4-Dimethyl-, 2,6-Dimethyl-, 3,4-Dimethyl- und 3,5-Dimethyl-pyridin, 5-Ethyl-2-methyl-pyridin, N-Methyl-piperidin, 4-Dimethylamino-pyridin, Diazabicyclooctan (DABCO), Diazabicyclononen (DBN) oder Diazabicycloundecen (DBU).

Alkalimetallhydroxide, wie Natrium- oder Kaliumhydroxid oder Alkalimetallalkoholate, wie Natriummethylat, werden als basische Reaktionshilfsmittel beim erfindungsgemäßen Verfahren besonders bevorzugt.

An Stelle der oben angegebenen basischen Reaktionshilfsmittel können auch die zur Herstellung der Ausgangsstoffe der Formel (II) einzusetzenden Aminoverbindungen der Formel (IV) - oben - als basische Reaktionshilfsmittel verwendet werden.

Als Verdünnungsmittel zur Durchführung des erfindungsgemäßen Verfahrens kommen neben Wasser die üblichen organischen Lösungsmittel in Betracht. Hierzu gehören insbesondere aliphatische, alicyclische oder aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan, Chloroform, Tetrachlormethan; Ether, wie Diethylether, Diisopropylether, t-Butyl-methylether, t-Pentyl-methylether, Dioxan, Tetrahydrofuran, Ethylenglykol-dimethyl- oder -diethylether, Diethylenglykol-dimethylether oder -diethylether; Ketone, wie Aceton, Butanon oder Methyl-isobutyl-keton; Nitrile, wie Acetonitril, Propionitril, Butyronitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäure-methylester, -ethylester, -n- oder -ipropylester, -n-, -i- oder -s-butylester, Sulfoxide, wie Dimethylsulfoxid; Alkohole, wie Methanol, Ethanol, n- oder i-Propanol, n-, i-, s- oder t-Butanol, Ethylenglykolmonomethylether oder -monoethylether, Diethylenglykolmonomethylether, Diethylenglykolmonoethylether; deren Gemische mit Wasser oder reines Wasser.

Wasser und Methanol werden als Verdünnungsmittel beim erfindungsgemäßen Verfahren besonders bevorzugt.

Die Reaktionstemperaturen können bei der Durchführung des erfindungsgemäßen Verfahrens in einem größeren Bereich variiert werden. Im Allgemeinen arbeitet man bei Temperaturen zwischen 0°C und 150°C, vorzugsweise bei Temperaturen zwischen 10°C und 120°C, insbesondere bei Temperaturen zwischen 20°C und 110°C.

Das erfindungsgemäße Verfahren wird im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Zur Durchführung des erfindungsgemäßen Verfahrens zur Herstellung der Verbindungen der Formel (I) setzt man pro Mol Hydrazincarbonsäureester der Formel (II) im Allgemeinen 1 bis 3 Mol, vorzugsweise 1,5 bis 2,5 Mol basisches Reaktionshilfsmittel ein.

In einer bevorzugten Ausführungsform des erfindunggemäßen Verfahrens wird der Hydrazincarbonsäureester der Formel (II) bei Raumtemperatur (ca. 20°C) in einem geeigneten Verdünnungsmittel aufgenommen und nach Zugabe eines basischen Reaktionshilfsmittels bei erhöhter Temperatur bis zum Ende der Umsetzung gerührt. Dann wird wieder auf Raumtemperatur (ca. 20°C) abgekühlt und mit einer starken Säure, wie z.B. Salzsäure oder Schwefelsäure angesäuert. Falls das Produkt der Formel (I) hierbei kristallin anfällt, kann es durch Absaugen isoliert werden. Andernfalls wird das Produkt der Formel (I) mit einem mit Wasser praktisch nicht mischbaren organischen Lösungsmittel, wie z.B. Essigsäureethylester extrahiert und von der organischen Phase wird dann - gegebenenfalls nach Trocknen und Filtrieren - das Lösungsmittel unter vermindertem Druck abdestilliert, wobei dann das Produkt der Formel (I) im Rückstand verbleibt.

In einer weiteren bevorzugten Ausführungsform des erfindungsgemäßen Verfahrens werden - sozusagen in einer "Ein-Topf-Variante" - die als Ausgangsstoffe benötigten Hydrazincarbonsäureester der Formel (II) durch Umsetzung von Hydrazincarbonsäureestern der Formel (III) mit Aminoverbindungen der Formel (IV) wie oben beschrieben hergestellt und anschließend ohne Zwischenisolierung - "in situ" - in die Triazolinone der Formel (I) umgewandelt.

Hierzu wird vorzugsweise zunächst ein Hydrazincarbonsäureester der Formel (III) bei mäßig erhöhter Temperatur - vorzugsweise zwischen 25°C und 35°C - zu einer Mischung aus überschüssiger Aminoverbindung der Formel (IV) - im Allgemeinen zwischen 2 und 10 Mol, vorzugsweise zwischen 2,5 und 5,0 Mol - und einem geeigneten Verdünnungsmittel - vorzugsweise Wasser - gegeben. Dann wird das Reaktionsgemisch bei weiter erhöhter Temperatur bis zum Ende der Umsetzung erhitzt und wie oben beschrieben aufgearbeitet.

Die nach dem erfindungsgemäßen Verfahren herzustellenden Triazolinone der Formel (I) können als Zwischenprodukte zur Herstellung von landwirtschaftlich nutzbaren Wirkstoffen verwendet werden (vgl. US-P-3 780 052, US-P-3 780 053, US-P-3 780 054 und EP-A-341489).

### Herstellungsbeispiele:

### Beispiel 1

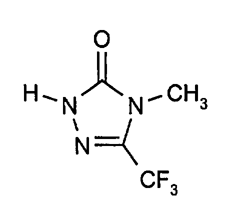

36 g (185 mMol) 2-(2,2,2-Trifluor-1-methylimino-ethyl)-hydrazin-1-carbonsäure-methylester werden in 200 ml Wasser suspendiert und nach Zugabe von 37,5 g 45 %iger wässriger Natronlauge (entsprechend 370 mMol NaOH) wird die Mischung 3 Stunden unter Rückfluss erhitzt. Nach Abkühlen auf Raumtemperatur (ca. 20°C) wird mit konz. Salzsäure angesäuert und dreimal mit je 100 ml Essigsäureethylester extrahiert. Die vereinigten organischen Extraktionslösungen werden mit Natriumsulfat getrocknet und filtriert. Vom Filtrat wird das Lösungsmittel im Wasserstrahlvakuum sorgfältig abdestilliert.

Man erhält 28,9 g (93,5% der Theorie) 4-Methyl-5-trifluormethyl-2,4-dihydro-3H-1,2,4-triazol-3-on als festen Rückstand.
¹H-NMR (DMSO-D₆, δ): 3,256 ppm (s, 3H), 12,655 ppm (s, 1H).

### Beispiel 2

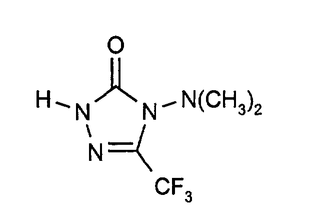

25,4 g (110 mMol) 2-(2,2,2-Trifluor-1-dimethylamino-ethyliden)-hydrazin-1-carbonsäure-methylester werden in 200 ml Wasser suspendiert und nach Zugabe von 19,8 g 45%iger wässriger Natronlauge (entsprechend 223 mMol NaOH) wird die Mischung 3 Stunden unter Rückfluss erhitzt. Nach Abkühlen auf Raumtemperatur (ca. 20°C) wird mit konz. Salzsäure angesäuert und das kristallin angefallene Produkt durch Absaugen isoliert.

Man erhält 19,1 g (89 % der Theorie) 4-Dimethylamino-5-trifluormethyl-2,4-dihydro-3H-1,2,4-triazol-3-on vom Schmelzpunkt 138°C.

### Beispiel 3

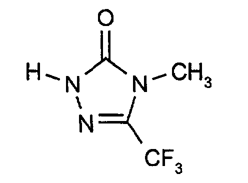

10,2 g (50 mMol) 2-(1-Chlor-2,2,2-trifluor-ethyliden)-hydrazin-1-carbonsäure-methylester werden portionsweise zu einer Mischung aus 50 ml Wasser und 22,5 g 34 %iger wässriger Methylamin-Lösung (entsprechend 250 mMol Methylamin) gegeben, wobei die Temperatur der Mischung zwischen 25°C und 35°C gehalten wird. Anschließend wird die Reaktionsmischung 2 Stunden unter Rückfluss erhitzt und dann - nach Abkühlen auf Raumtemperatur (ca. 20°C) mit konz. Salzsäure angesäuert. Dann wird dreimal mit Essigsäureethylester extrahiert. Die vereinigten Extraktionslösungen werden mit Natriumsulfat getrocknet und filtriert. Vom Filtrat wird dann das Lösungsmittel im Wasserstrahlvakuum sorgfältig abdestilliert.

Man erhält 7,3 g (85 % der Theorie) 4-Methyl-5-trifluormethyl-2,4-dihydro-3H-1,2,4-triazol-3-on als festen Rückstand.

(Gehalt: 97,0 %).

Analog zu den Beispielen 1 bis 3 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

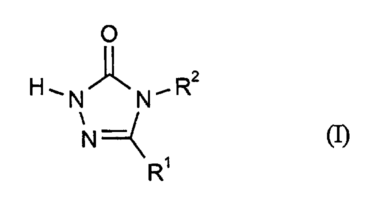

**Tabelle 1:**

| Beispiele für die erfindungsgemäß erhaltenen Verbindungen der Formel (I) | | | | |
|---|---|---|---|---|
| **Beisp.- Nr.** | **R**^{**1**} | **R**^{**2**} | **Ausbeute (% der Theorie)** | **Physikal. Daten** |
| 4 | CF₃ | (CH₂)₂OCH₃ | 92 | n_{D}²⁰=1,4290 |
| 5 | CF₃ | (CH₂)₃OCH₃ | 90 | n_{D}²⁰=l,4320 |
| 6 | CHF₂ | CH₃ | | |
| 7 | CF₃ | H | 70 | Fp.: 198°C |

### Ausgangsstoffe der Formel (II)

### Beispiel (II-1)

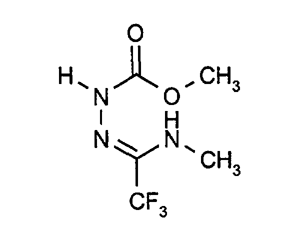

39,6 g (185 mMol) 2-(1-Chlor-2,2,2-trifluor-ethyliden)-hydrazin-1-carbonsäure-methylester werden in 220 ml Dioxan gelöst und zu dieser Lösung werden bei 20°C 50 g einer 34,5 %igen wässrigen Lösung von Methylamin (entsprechend 556 mMol Methylamin) innerhalb von 30 Minuten tropfenweise gegeben, wobei die Innentemperatur zwischen 25°C und 35°C gehalten wird. Die Mischung wird dann noch 2 Stunden bei ca. 30°C gerührt und anschließend im Wasserstrahlvakuum eingeengt. Der Rückstand wird mit 150 ml Wasser verrührt und das kristallin angefallene Produkt durch Absaugen isoliert.

Man erhält 36 g (98 % der Theorie) 2-(2,2,2-Trifluor-1-methylimino-ethyl)-hydrazin-1-carbonsäure-methylester.
¹H-NMR (DMSO-D₆, δ): 2,817 ppm (m, 3H), 3,641 ppm (s, 3H), 6,483 ppm (br. s, 1H), 9,527 ppm (s, 1H).

### Beispiel (II-2)

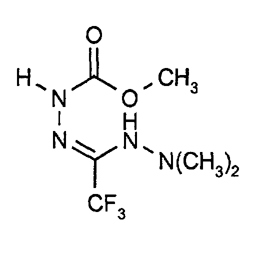

8,06 g (134 mMol) N,N-Dimethyl-hydrazin und 17 ml (122 mMol) Triethylamin werden in 120 ml Acetonitril vorgelegt und bei einer Innentemperatur von ca. 55°C wird eine Lösung von 25 g (122 mMol) 2-(1-Chlor-2,2,2-trifluor-ethyliden)-hydrazin-1-carbonsäure-methylester in 30 ml Acetonitril unter Rühren tropfenweise dazu gegeben. Die Mischung wird dann noch eine Stunde bei 60°C gerührt und anschließend im Wasserstrahlvakuum eingeengt. Der Rückstand wird in Essigsäureethylester aufgenommen, mit Wasser gewaschen, mit Natriumsulfat getrocknet und filtriert. Das Filtrat wird im Wasserstrahlvakuum eingeengt, der Rückstand durch Digerieren mit Diisopropylether zur Kristallisation gebracht und das kristalline Produkt durch Absaugen isoliert.

Man erhält 16,1 g (58 % der Theorie) 2-(2,2,2-Trifluor-1-(N,N-dimethylhydrazino)-ethyl)-hydrazin-1-carbonsäure-methylester vom Schmelzpunkt 124°C.

Analog zu den Beispielen (II-1) und (II-2) können beispielsweise auch die in der nachstehenden Tabelle 2 aufgeführten Verbindungen der Formel (II) hergestellt werden.

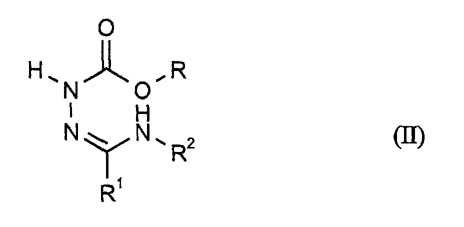

**Tabelle 2:**

| Beispiele für die Verbindungen der Formel (II) | | | | | |
|---|---|---|---|---|---|
| **Beisp.- Nr.** | **R**^{**1**} | **R**^{**2**} | **R** | **Ausbeute (% derTheorie)** | **Schmelz- punkt (°C)** |
| II-3 | CF₃ | CH₃ | CH₃ | 88 | 213 |
| II-4 | CF₃ | (CH₂)₂OCH₃ | CH₃ | 68 | 150 |
| II-5 | CF₃ | (CH₂)₃OCH₃ | CH₃ | 84 | 148 |
| II-6 | CF₃ | OH | CH₃ | 63 | 183 |
| II-7 | CF₃ | OCH₃ | CH₃ | 27 | 104 |
| II-8 | CHF₂ | CH₃ | CH₃ | | |

### Vorprodukte der Formel (III):

### Beispiel (III-1)

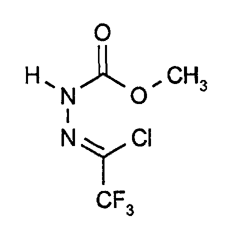

65,1 g (350 mMol) 2-Trifluoracetyl-hydrazin-1-carbonsäure-methylester und 66,2 g (375 mMol) Benzolsulfonsäurechlorid werden in 450 ml Aceton vorgelegt und bei einer Innentemperatur zwischen 50°C und 55°C wird unter Rühren eine Lösung von 48,7 ml (350 mMol) Triethylamin in 50 ml Aceton zugetropft. Nach einstündigem Rühren bei 50°C bis 55°C werden 2,5 ml Triethylamin zur Reaktionsmischung gegeben und das Rühren wird weitere 30 Minuten fortgesetzt. Nach Abkühlen mit einem Eisbad wird das kristallin angefallene Triethylammoniumchlorid durch Absaugen abgetrennt. Das Filtrat wird im Wasserstrahlvakuum eingeengt, der Rückstand in 300 ml Essigsäureethylester aufgenommen, diese Lösung dreimal mit je 100 ml Wasser gewaschen, mit Natriumsulfat getrocknet und filtriert. Vom Filtrat wird das Lösungsmittel im Wasserstrahlvakuum sorgfältig abdestilliert.

Man erhält 72,5 g (Gehalt: 92,2 %, Ausbeute: 93 % der Theorie) 2-(1-Chlor-2,2,2-trifluor-ethyliden)-hydrazin-1-carbonsäure-methylester als festes Produkt.
¹H-NMR (DMSO-D₆, δ): 3,78 ppm (s, 3H), 11,59 ppm (s, 1H).

Analog Beispiel (III-1) können beispielsweise auch die in der nachstehenden Tabelle 3 aufgeführten Verbindungen der Formel (III) hergestellt werden.

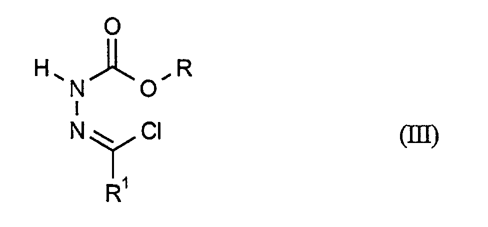

**Tabelle 3:**

| Beispiele für die Verbindungen der Formel (III) | | | | |
|---|---|---|---|---|
| **Bsp.- Nr.** | **R**^{**1**} | **R** | **Ausbeute (% der Theorie)** | **Schmelz- punkt (°C)** |
| III-2 | CHF₂ | CH₃ | | |

### Vorprodukte der Formel (V):

### Beispiel (V-1)

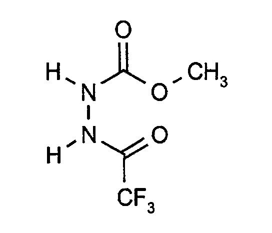

121,5 g (1,28 Mol) Carbazinsäure-methylester (Hydrazincarbonsäure-methylester) werden in 1200 ml Diethylether vorgelegt und bei 0°C werden 310 g (1,48 Mol) Trifluoressigsäureanhydrid innerhalb von 2 Stunden tropfenweise dazu gegeben. Dann wird die Mischung noch ca. 90 Minuten bei 0°C bis 20°C gerührt, anschließend im Wasserstrahlvakuum eingeengt, der Rückstand mit 500 ml Toluol verrührt und das kristalline Produkt durch Absaugen isoliert.

Man erhält 219 g (Gehalt: 94,4 %, Ausbeute: 94 % der Theorie) 2-Trifluoracetylhydrazin-1-carbonsäure-methylester vom Schmelzpunkt 105°C.
¹H-NMR (DMSO-D₆, δ): 3,647 ppm (s, 3H), 9,649 ppm (s, 1H), 11,429 ppm (s, 1H).

### Beispiel (V-2)

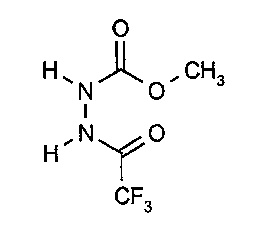

18,5 g (0,20 Mol) Carbazinsäure-methylester (Hydrazincarbonsäure-methylester) werden in 150 ml Toluol vorgelegt und bei einer Innentemperatur von 80°C werden 23,1 ml (0,30 Mol) Trifluoressigsäure innerhalb von 35 Minuten tropfenweise dazu gegeben. Die Mischung wird dann weitere 30 Minuten bei 80°C gerührt, anschließend eine Stunde am Wasserabscheider erhitzt und dann im Wasserstrahlvakuum eingeengt. Der Rückstand mit 500 ml Toluol verrührt und das kristalline Produkt durch Absaugen isoliert.

Man erhält 36,4 g (Gehalt: 90,1 %, Ausbeute: 88,2 % der Theorie) 2-Trifluoracetylhydrazin-1-carbonsäure-methylester vom Schmelzpunkt 105°C.

## Patentansprüche

1. Verfahren zur Herstellung von Triazolinonen der allgemeinen Formel (I) 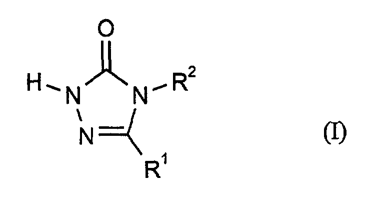 in welcher
R¹ für zweifach oder dreifach durch Fluor substituiertes Methyl steht und
R² für jeweils gegebenenfalls durch Fluor, Chlor, Cyano, Methoxy oder Ethoxy substituiertes Methyl, Ethyl, n- oder i-Propyl, n-, i- oder s-Butyl, Allyl, Propargyl, Methoxy, Ethoxy, n- oder i-Propoxy, n-, i- oder s-Butoxy, Methylamino, Ethylamino, n- oder i-Propylamino, n-, i- oder s-Butylamino, Dimethylamino oder Diethylamino oder für jeweils gegebenenfalls durch Fluor, Chlor, Brom, Cyano, Methyl, Ethyl, n- oder i-Propyl, Methoxy-carbonyl oder Ethoxycarbonyl substituiertes Cyclopropyl, Cyclobutyl, Cyclopentyl, Cyclohexyl, Phenyl oder Benzyl steht,
**dadurch gekennzeichnet, daß** man Hydrazincarbonsäureester der allgemeinen Formel (II) 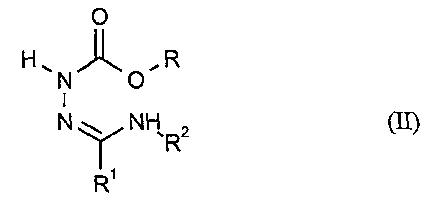 in welcher
R¹ und R² die oben angegebenen Bedeutungen haben und
R für gegebenenfalls durch Halogen oder C₁-C₄-Alkoxy substituiertes Alkyl mit 1 bis 4 Kohlenstoffatomen oder für gegebenenfalls durch Halogen oder C₁-C₄-Alkyl substituiertes Phenyl steht,
- und/oder zu den Verbindungen der Formel (II) tautomere Verbindungen -
in Gegenwart eines basischen Reaktionshilfsmittels und in Gegenwart eines Verdünnungsmittels bei Temperaturen zwischen 0°C und 150°C umsetzt.

2. Hydrazincarbonsäureester der allgemeinen Formel (II) 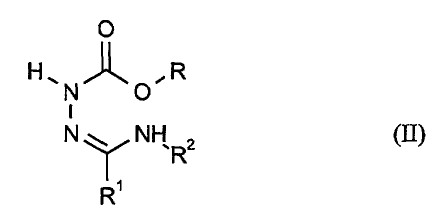 in welcher
R, R¹ und R² die in Anspruch 1 genannte Bedeutung haben.

3. Verfahren gemäß Anspruch 1, **dadurch gekennzeichnet, dass** die Hydrazincarbonsäureester der allgemeinen Formel (II), 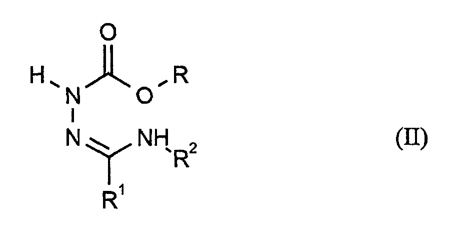 in welcher
R, R¹ und R² die in Anspruch 1 genannten Bedeutungen haben,
in einem zuvor durchgeführten Reaktionsschritt dadurch hergestellt werden, dass entsprechende Hydrazincarbonsäureester der allgemeinen Formel (III), 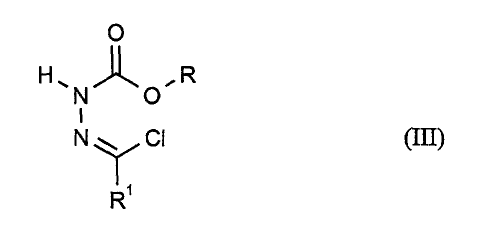 in welcher
R und R¹ die oben angegebenen Bedeutungen haben,
mit Aminoverbindungen der allgemeinen Formel (IV)
H₂N-R² (IV)
in welcher
R² die oben angegebene Bedeutung hat,
gegebenenfalls in Gegenwart eines Säureakzeptors und gegebenenfalls in Gegenwart eines Verdünnungsmittels bei Temperaturen zwischen 0°C und 100°C umgesetzt werden.

4. Verfahren gemäß Anspruch 3, **dadurch gekennzeichnet, dass** die Hydrazincarbonsäureestern der allgemeinen Formel (III), 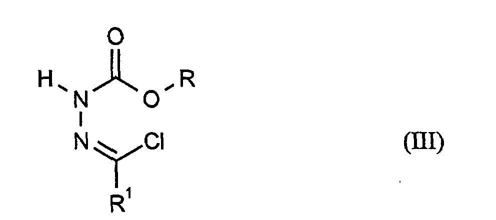 in welcher
R, R¹ und R² die in Anspruch 3 genannten Bedeutungen haben,
in einem zuvor durchgeführten Reaktionsschritt dadurch hergestellt werden, dass entsprechende Hydrazincarbonsäureester der allgemeinen Formel (V) 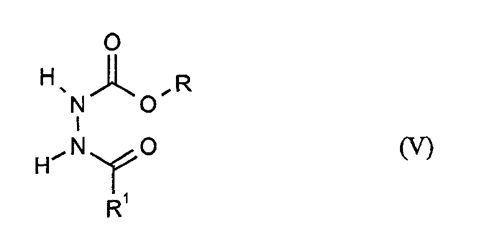 in welcher
R und R¹ die oben angegebenen Bedeutungen haben,
mit einem Sulfonsäurechlorid gegebenenfalls in Gegenwart eines Säureakzeptors und gegebenenfalls in Gegenwart eines Verdünnungsmittels bei Temperaturen zwischen 0°C und 100°C umgesetzt werden.

5. Verfahren gemäß Anspruch 4, **dadurch gekennzeichnet, dass** die Hydrazincarbonsäureester der allgemeinen Formel (Va), 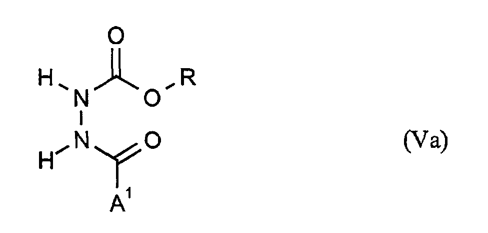 in welcher
A¹ für Difluormethyl oder Trifluormethyl steht und
R wie in Anspruch 4 definiert ist,
in einem zuvor durchgeführten Reaktionsschritt dadurch hergestellt werden, dass Hydrazincarbonsäureester der Formel (VI), 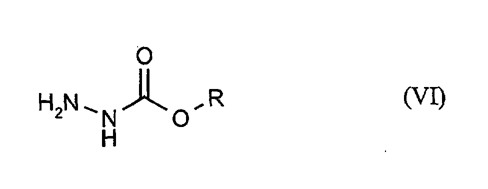 in welcher
R die oben angegebene Bedeutung hat,
mit Carbonsäuren oder deren Derivaten der allgemeinen Formel (VII),
A¹-CO-X (VII)
in welcher
A¹ die oben angegebene Bedeutung hat und
X für Hydroxy, Halogen oder die Gruppierung -O-CO-A¹ steht,
gegebenenfalls in Gegenwart eines Verdünnungsmittels bei Temperaturen zwischen 0°C und 150°C umgesetzt werden.

## Claims

1. Process for preparing triazolinones of the general formula (I) 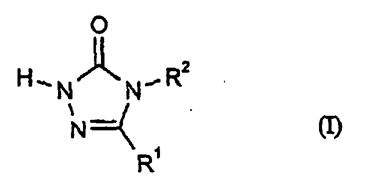 in which
R¹ represents methyl which is di- or trisubstituted by fluorine, and
R² represents methyl, ethyl, n- or i-propyl, n-, i- or s-butyl, allyl, propargyl, methoxy, ethoxy, n- or i-propoxy, n-, i- or s-butoxy, methylamino, ethylamino, n- or i-propylamino, n- or i- or s-butylamino, dimethylamino or diethylamino which are in each case optionally substituted by fluorine, chlorine, cyano, methoxy or ethoxy, or represents cyclopropyl, cyclobutyl, cyclopentyl, cyclohexyl, phenyl or benzyl which are in each case optionally substituted by fluorine, chlorine, bromine, cyano, methyl, ethyl, n- or i-propyl, methoxycarbonyl or ethoxycarbonyl,
**characterized in that** hydrazinecarboxylic esters of the general formula (II) 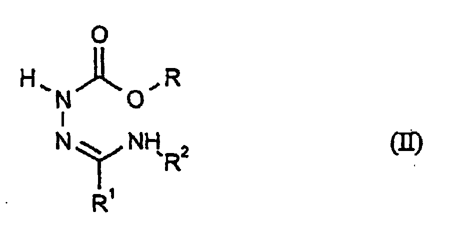 in which
R¹ and R² have the abovementioned meanings, and
R represents alkyl which is optionally substituted by halogen or C₁-C₄-alkoxy, or represents phenyl which is optionally substituted by halogen or C₁-C₄-alkyl,
- and/or compounds which are tautomeric to the compounds of the formula (II) -
are reacted in the presence of a basic reaction auxiliary and in the presence of a diluent, at temperatures of between 0°C and 150°C.

2. Hydrazinecarboxylic esters of the general formula (II) 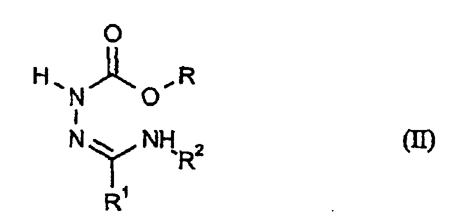 in which
R, R¹ and R² have the meaning given in Claim 1.

3. Process according to Claim 1, **characterized in that** the hydrazinecarboxylic esters of the general formula (II) 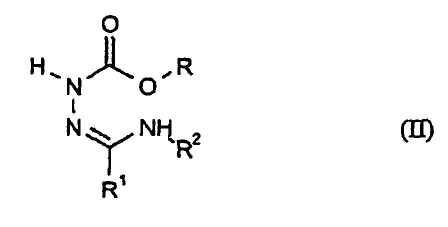 in which
R, R¹ and R² have the meanings given in Claim 1,
are prepared in a preceding reaction step by reacting corresponding hydrazinecarboxylic esters of the general formula (III) 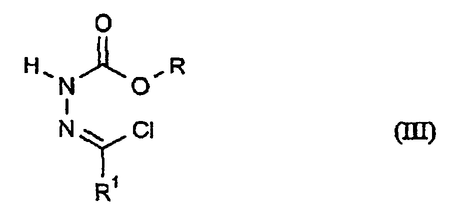 in which
R and R¹ have the abovementioned meanings,
with amino compounds of the general formula (IV)
H₂N-R² (IV)
in which
R² has the abovementioned meaning,
where appropriate in the presence of an acid acceptor and where appropriate in the presence of a diluent, at temperatures of between 0°C and 100°C.

4. Process according to Claim 3, **characterized in that** the hydrazinecarboxylic esters of the general formula (III) 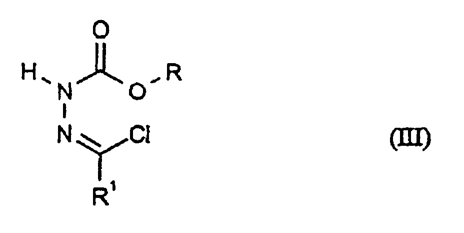 in which
R, R¹ and R² have the meanings given in Claim 3,
are prepared in a preceding reaction step by reacting corresponding hydrazinecarboxylic esters of the general formula (V) 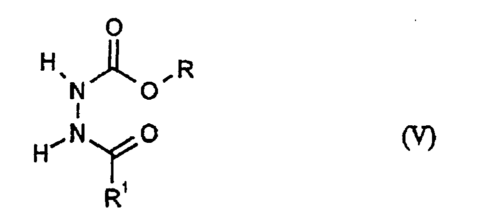 in which
R and R¹ have the abovementioned meanings,
with a sulphonyl chloride, where appropriate in the presence of an acid acceptor and where appropriate in the presence of a diluent, at temperatures of between 0°C and 100°C.

5. Process according to Claim 4, **characterized in that** the hydrazinecarboxylic esters of the general formula (Va) 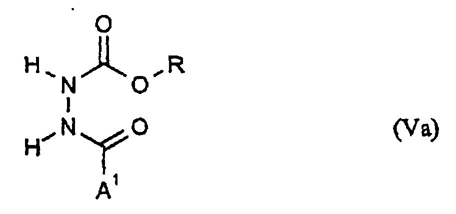 in which
A¹ represents difluoromethyl or trifluoromethyl, and
R is as defined in Claim 4, are prepared in a preceding reaction step by reacting hydrazinecarboxylic esters of the formula (VI) 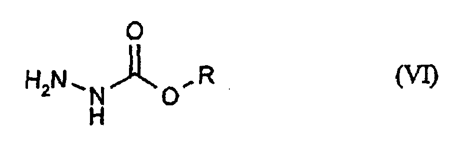
in which
R has the abovementioned meaning,
with carboxylic acids, or their derivatives, of the general formula (VII)
A¹-CO-X (VII)
in which
A¹ has the abovementioned meaning, and
X represents hydroxyl, halogen or the group -O-CO-A¹,
where appropriate in the presence of a diluent, at temperatures of between 0°C and 150°C.

## Revendications

1. Procédé pour la préparation de triazolinones répondant à la formule générale (I) 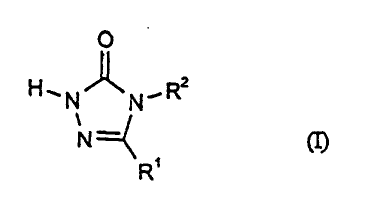 dans laquelle
R¹ représente un groupe méthyle portant deux ou trois substituants fluoro et
R² représente un groupe méthyle, un groupe éthyle, un groupe n- ou i-propyle, un groupe n-, i- ou s-butyle, un groupe allyle, un groupe propargyle, un groupe méthoxy, un groupe éthoxy, un groupe n- ou i-propoxy, un groupe n-, i- ou s-butoxy, un groupe méthylamino, un groupe éthylamino, un groupe n- ou i-propylamino, un groupe n-, i- ou s-butylamino, un groupe diméthylamino ou un groupe diéthylamino, chacun de ces groupes portant le cas échéant un ou plusieurs substituants fluoro, chloro, cyano, méthoxy ou éthoxy, ou bien représente un groupe cyclopropyle, un groupe cyclobutyle, un groupe cyclopentyle, un groupe cyclohexyle, un groupe phényle ou un groupe benzyle, chacun de ces groupes portant le cas échéant un ou plusieurs substituants fluoro, chloro, bromo, cyano, méthyle, éthyle, n- ou i-propyle, méthoxycarbonyle ou éthoxycarbonyle,
**caractérisé en ce qu'**on fait réagir des esters hydrazinecarboxyliques répondant à la formule générale (II) 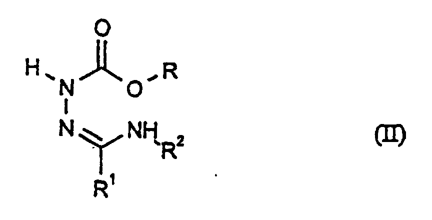 dans laquelle
R¹ et R² ont les significations indiquées ci-dessus et
R représente un groupe alkyle contenant de 1 à 4 atomes de carbone portant le cas échéant un ou plusieurs substituants halogéno ou alcoxy en C₁-C₄, ou représente un groupe phényle portant le cas échéant un ou plusieurs substituants halogéno ou alkyle en C₁-C₄,
- et/ou pour obtenir les composés tautomères vis-à-vis des composés répondant à la formule (II) -
en présence d'un adjuvant réactionnel basique et en présence d'un diluant à des températures entre 0 °C et 150 °C.

2. Esters hydrazinecarboxyliques répondant à la formule générale (II) 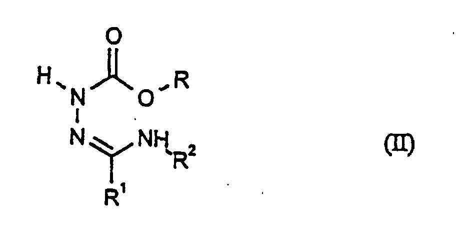 dans laquelle
R, R¹ et R² ont la signification indiquée à la revendication 1.

3. Procédé selon la revendication 1, **caractérisé en ce qu'**on prépare les esters hydrazinecarboxyliques répondant à la formule générale (II) 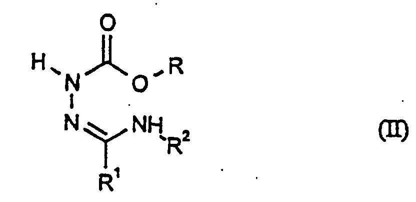 dans laquelle
R, R¹ et R² ont les significations indiquées à la revendication 1,
dans une étape réactionnelle mise en oeuvre au préalable de telle sorte que l'on fait réagir des esters hydrazinecarboxyliques correspondants répondant à la formule générale (III) 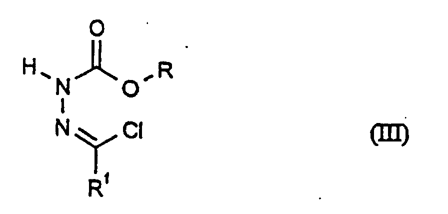 dans laquelle
R et R¹ ont les significations indiquées ci-dessus,
avec des composés amino répondant à la formule générale (IV)
H₂N-R² (IV)
dans laquelle
R² a la signification indiquée ci-dessus,
le cas échéant, en présence d'un agent neutralisant les acides et le cas échéant, en présence d'un diluant à des températures entre 0 °C et 100 °C.

4. Procédé selon la revendication 3, **caractérisé en ce qu'**on prépare les esters hydrazinecarboxyliques répondant à la formule générale (III) 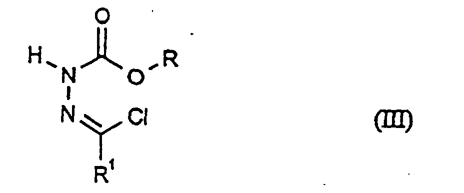 dans laquelle
R, R¹ et R² ont les significations indiquées à la revendication 3,
dans une étape réactionnelle mise en oeuvre au préalable de telle sorte que l'on fait réagir des esters hydrazinecarboxyliques correspondants répondant à la formule générale (V) 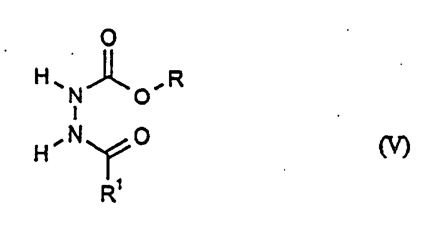 dans laquelle
R et R¹ ont les significations indiquées ci-dessus,
avec un chlorure d'acide sulfonique, le cas échéant en présence d'un agent neutralisant les acides et le cas échéant en présence d'un diluant, à des températures entre 0 °C et 100 °C.

5. Procédé selon la revendication 4, **caractérisé en ce qu'**on prépare les esters hydrazinecarboxyliques répondant à la formule générale (Va) 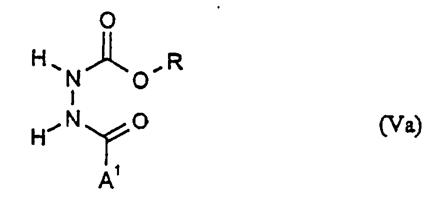 dans laquelle
A¹ représente un groupe difluorométhyle ou trifluorométhyle et
R est tel que défini à la revendication 4,
dans une étape réactionnelle mise en oeuvre au préalable de telle sorte que l'on fait réagir des esters hydrazinecarboxyliques correspondants répondant à la formule générale (VI) 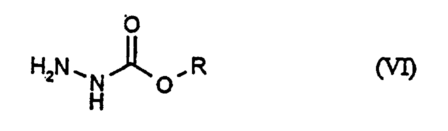 dans laquelle
R a la signification indiquée ci-dessus,.
avec des acides carboxyliques ou leurs dérivés répondant à la formule générale (VII)
A¹-CO-X (VII)
dans laquelle
A¹ a la signification indiquée ci-dessus et
X représente un groupe hydroxyle, un atome d'halogène ou encore le groupement -O-CO-A¹
le cas échéant en présence d'un diluant à des températures entre 0 °C et 150 °C.
